# EUROPEAN PATENT SPECIFICATION

(11) **EP 2 102 335 B1**
(45) Date of publication and mention of the grant of the patent: **22.02.2012**
(21) Application number: 08701217.5
(22) Date of filing: 03.01.2008
(51) Int. Cl.: C12N 9/64, C07K 14/745, C07K 1/20

(54) **PURIFICATION OF FACTOR XI**
FAKTOR-XI-REINIGUNG
PURIFICATION DE FACTEUR XI

(30) Priority: 04.01.2007 EP 07100123; 04.01.2007 US 878509 P
(43) Date of publication of application: 23.09.2009
(73) Proprietor: Crucell Holland B.V., 2333 CN Leiden (NL)
(72) Inventor: MARKUS, Paul Henri, NL-2333 CN Leiden (NL)
(74) Representative: Manten, Annemieke
(86) International application number: PCT/EP2008/050035
(87) International publication number: WO 2008/081025

(56) References cited:
- EP-A1- 1 026 172
- WO-A-2004/041979
- WO-A-2006/110277
- WO-A1-02/15927
- US-A- 5 252 217
- BURTON S C ET AL: "Hydrophobic charge induction chromatography: salt independent protein adsorption and facile elution with aqueous buffers" JOURNAL OF CHROMATOGRAPHY A, ELSEVIER, AMSTERDAM, NL, vol. 814, no. 1-2, 24 July 1998 (1998-07-24), pages 71-81, XP004145777 ISSN: 0021-9673 cited in the application
- BURTON S ET AL: "Salt-independent adsorption chromatography: new broad-spectrum affinity methods for protein capture" JOURNAL OF BIOCHEMICAL AND BIOPHYSICAL METHODS, AMSTERDAM, NL, vol. 49, 2001, pages 275-287, XP002987338 ISSN: 0165-022X
- WEATHERLY G T ET AL: "Initial purification of recombinant botulinum neurotoxin fragments for pharmaceutical production using hydrophobic charge induction chromatography" JOURNAL OF CHROMATOGRAPHY A, ELSEVIER, AMSTERDAM, NL, vol. 952, no. 1-2, 5 April 2002 (2002-04-05), pages 99-110, XP004346350 ISSN: 0021-9673
- MASHIKO H ET AL: "PURIFICATION OF FACTOR XI AND SOME PROPERTIES OF ACTIVATED FACTOR XI FROM PORCINE PLASMA" AGENTS AND ACTIONS SUPPLEMENTS, BIRKHAEUSER VERLAG, BASEL, CH, vol. 38, no. PART 2, 1992, pages 249-256, XP009084254 ISSN: 0379-0363
- BURNOUF T ET AL: "Affinity chromatography in the industrial purification of plasma proteins for therapeutic use" JOURNAL OF BIOCHEMICAL AND BIOPHYSICAL METHODS, AMSTERDAM, NL, vol. 49, no. 1-3, 30 October 2001 (2001-10-30), pages 575-586, XP002353092 ISSN: 0165-022X
- SCHWARTZ W ET AL: "Comparison of hydrophobic charge induction chromatography with affinity chromatography on protein A for harvest and purification of antibodies" JOURNAL OF CHROMATOGRAPHY A, ELSEVIER, AMSTERDAM, NL, vol. 908, no. 1-2, 26 January 2001 (2001-01-26), pages 251-263, XP004314138 ISSN: 0021-9673 cited in the application
- BURTON S C ET AL: "Preparation of chromatographic matrices by free radical addition ligand attachment to allyl groups" JOURNAL OF CHROMATOGRAPHY A, ELSEVIER, AMSTERDAM, NL, vol. 796, no. 2, 20 February 1998 (1998-02-20), pages 273-282, XP004110807 ISSN: 0021-9673
- SUN YUEHUI ET AL: "Identification of a factor IX binding site on the third apple domain of activated factor XI" JOURNAL OF BIOLOGICAL CHEMISTRY, vol. 271, no. 46, 1996, pages 29023-29028, XP002435764 ISSN: 0021-9258 cited in the application

## Description

The invention relates to the field of proteins, in particular purification of proteins. More in particular, the invention relates to the purification of blood coagulation Factor XI.

### Background of the invention

Factor XI is a protein involved in the blood coagulation cascade, and this protein may for instance be used in hemostatic treatment, see e.g. WO 2005/049070. Factor XI deficiency leads to impairment of an amplification loop in the blood coagulation cascade, resulting in Hemophilia C, which is a mild bleeding disorder and bleeding is typically induced by surgery or trauma. Hemophilia C patients can be treated with preparations containing Factor XI to compensate for their deficiency. Factor XI comprises 607 amino acid residues and circulates in plasma at a concentration of about 4-6 µg/ml (30 nM) as a disulfide-linked homo-dimer with a Mw of about 160 kD. Each monomer contains 4 so-called apple-domains and one serine protease domain. Factor XI can be activated to Factor XIa by Factor XIIa, thrombin and by Factor XIa itself. It is localized in plasma (in complex with cofactor high molecular weight kininogen) and found on platelets.

For several purposes, for instance when Factor XI is to be administered to subjects for therapeutic purposes, it is desired to purify Factor XI from a biological fluid in which it is contained. Factor XI could for instance be obtained from blood plasma, or by recombinant expression. Recombinant expression of Factor XI has been described, see e.g. Kemball-Cook et al (1994), Gene 139: 275; Meijers et al (1992), Blood 79: 1435. Purification of Factor XI has also been reported in the art, see e.g. Bouma and Griffin (1977), J Biol Chem 252: 6432; Fujikawa et al (1986), Biochem. 25: 2417.

US patent 5,252,217 describes a method for obtaining a Factor XI concentrate having high specific activity and suitable for therapeutic use, starting from human blood plasma, comprising a first step of filtration-adsorption to specific negatively charged filters, desorption of the filters followed by a second step involving chromatography on a cation exchange resin.

WO 2005/049070 and WO 2006/128497 mention methods for purification of Factor XI from other biological material, comprising subjecting the material to cation-exchange chromatography, hydrophobic interaction chromatography or hydroxyapatite chromatography, and in preferred embodiments to a combination of these three types of chromatography in the given order.

Hydrophobic Charge Induction Chromatography (HCIC) is a relatively new chromatography method, based on the pH-dependent behaviour of ionizable, dual-mode ligands, described by Burton and Harding (1998), J. Chromatogr. A 814: 71. It has been particularly advocated for the purification of antibodies, see e.g. Schwartz et al (2001), J Chromatogr. A 908: 251. A HCIC matrix carrying an antibody-selective ligand, 4-Mercapto-Ethyl-Pyridine (4-MEP), is commercially available under the name HyperCel, and is mentioned to be specifically designed for purification of antibodies, see datasheet of MEP HyperCel on http://www.pall.com/datasheet_biopharm_35677.asp. WO 2005/014621 describes the use of HCIC for purification of a non-immunoglobulin protein comprising an immunoglobulin-like domain.

There remains a need in the art for further possibilities to purify Factor XI. It is the object of the present invention to provide alternative methods for purification of Factor XI.

### Summary of the invention

The invention provides a method for purifying or capturing coagulation Factor XI from a biological fluid, comprising the steps of: a) contacting the biological fluid containing coagulation Factor XI with an Hydrophobic Charge Induction Chromatography (HCIC) material; b)washing the HCIC material with a solution having a pH between 5.5 and 12; and c) eluting the coagulation Factor XI by treating the HCIC material with a solution having a pH that is lower than 5.5.

In one embodiment, the HCIC material comprises 4-Mercapto-Ethyl-Pyridine (4-MEP), for instance linked to a cellulose matrix. The HCIC material for instance may be MEP-HyperCel.

In preferred embodiments, step a) is carried out at a pH between 6 and 9.

In preferred embodiments, step b) comprises washing with a solution having a pH between 6 and 9.

In preferred embodiments, step c) is carried out using a solution having a pH between 5.5 and 3.

In preferred embodiments, the method further comprises an anion exchange step, wherein impurities are bound to an anion exchange material and Factor XI is not bound. Such anion exchange step is preferably before the HCIC step, but alternatively may be after the HClC step of the invention.

In further embodiments, the method further comprises a step of cation exchange chromatography, wherein Factor XI is bound to the cation exchange material and subsequently eluted therefrom.

In further embodiments, the method further comprises a step of size-exclusion chromatography.

In further embodiments, the method further comprises another chromatography step, such as hydroxyapatite chromatography, hydrophobic interaction chromatography, affinity chromatography, e.g. using an antibody or other ligand specific for Factor XI.

In certain embodiments, the biological fluid is chosen from blood plasma, cell culture supernatants, cell-conditioned culture medium and fractions derived from an earlier chromatographic separation step.

In certain embodiments, the coagulation Factor XI has been recombinantly expressed.

The invention further provides the use of a Hydrophobic Charge Induction Chromatography (HCIC) material for the purification or capturing of coagulation Factor XI from a biological fluid.

### Brief description of the Figures

FIG. 1. Gradient profile of Factor XI preparation on a 1 ml MEP HyperCel column. A 7-step gradient (pH 6-3, citrate buffer, 50 mM NaCl) is shown. Factor XI peaks are indicated with arrows. See example 3 for details.
FIG. 2. Western blot of selected fractions from run shown in Fig. 1.
   Left panel. NR: non-reduced conditions. 1: starting material, 2: marker (Pre stained See Blue), 3: FT (flow through: unbound material), 4: peak A, 5: peak B, 6: peak C, 7: peak D, 8: peak E, 9: peak F, 10: control (Factor XI Kordia, 0.68 mg/ml 1:100). R: reduced conditions. 1: marker, 2: starting material, 3: FT, 4: peak A, 5: peak B, 6: peak C, 7: peak D, 8: peak E, 9: peak F, 10: control (Factor XI Kordia, 0.68 mg/ml 1:100).

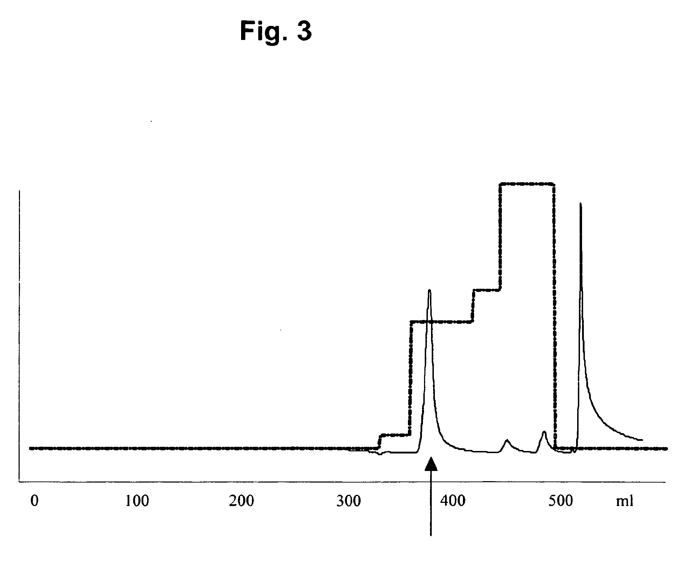

FIG. 3. Gradient profile of Factor XI preparation from a serum-free culture medium on a 1 ml MEP HyperCel column. See example 4 for details. The Factor XI peak is indicated by an arrow.

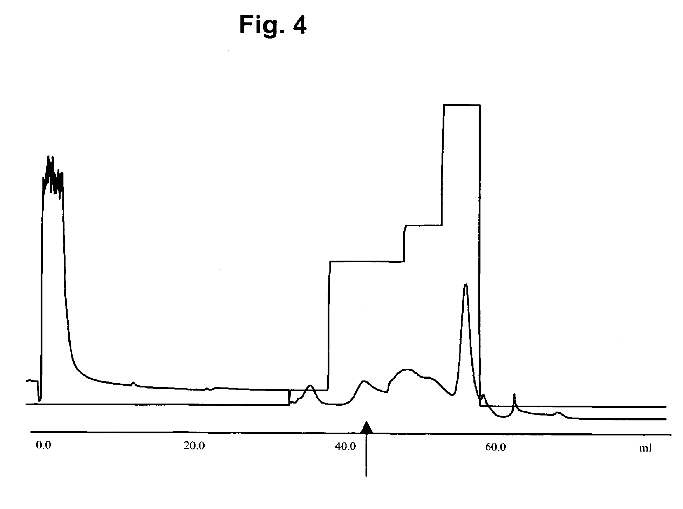

FIG. 4. Gradient profile of the flow through fraction of a protein G column of a Factor XI preparation from a DEAE treated human plasma, on a 1 ml MEP HyperCel column. See example 5 for details. The Factor XI peak is indicated by an arrow.

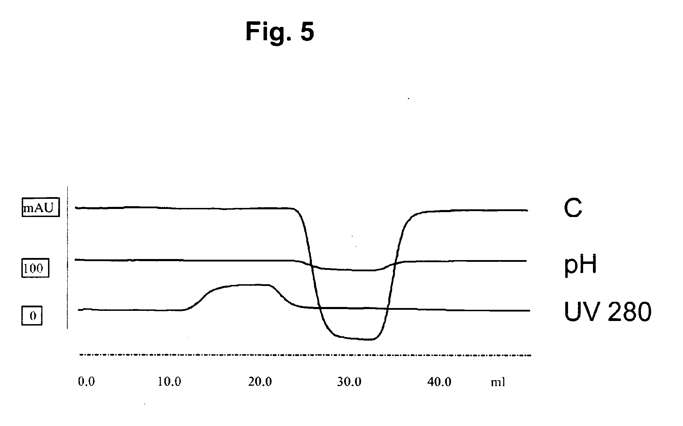

FIG. 5. Profile of 10 ml HCIC-purified Factor XI fraction on a 30 ml Sephadex 75 column, for polishing and buffer exchange. C: conductivity. See example 6 for details.

### Detailed description of the invention

MEP HyperCel is a Hydrophobic Charge Induction Chromatography (HCIC) resin that is commercially available from Pall Filtron, and is especially designed for the primary capture and purification of antibodies (datasheet of MEP HyperCel on http://www.pall.com/datasheet_biopharm_35677.asp). At neutral pH, hydrophobic capture occurs in HCIC resin by both an aliphatic-hydrophobic spacer and a neutral (uncharged) pyridine ring. In contrast to hydrophobic interaction (HI) chromatography, adsorption of antibodies from cell culture supernatants on HCIC resin is accomplished without the need of any pH or ionic strength adjustment. Once the pH is lowered from pH 7.2 to pH 4, the pyridine ring in the resin and the bound antibody become positively charged, due to charge repulsion, the immunoglobulin detaches and elutes from the column.

The present invention is based on the surprising finding that Factor XI binds very well to this HCIC material, and that it can also be eluted under mild conditions with a weakly acidic buffer from this resin with a good yield and a good purity. This is surprising because Factor XI has a completely different structure than immunoglobulins and does not contain immunoglobulin-like domains. Batchwise and column purifications using MEP HyperCel were carried out resulting in Factor XI preparations that did not show activation to Factor XIa after the capture step, and could be activated in a clotting assay. Hence, the purified Factor XI remained active as desired, and did not show undesired activation during the purification steps using HCIC.

An HCIC resin that can be used according to the present invention is hydrophobic (mainly uncharged) at pH 7-8.5 and is positively charged (significantly ionised) at pH 3-4. Such a resin allows the binding or adsorption of Factor XI based on mild hydrophobic interaction, achieved without the necessity to add lyotropic (e.g. ammonium sulphate) or other salts to a biological fluid, and elution or desorption based on charge repulsion under weakly acidic conditions.

An HCIC resin as used herein usually comprises a ligand to which the Factor XI binds, and a solid support to which the ligand is linked. A suitable ligand for HCIC for purifying Factor XI according to the invention is 4-Mercapto-Ethyl-Pyrazine (4-MEP). Other ligands with similar properties should also work, e.g. weak base pyridyl or imidazolyl ligands such as for instance 4-mercaptopyridine, mercaptomethylimidazole, 2-mercaptobenzimidazole, aminoethylbenzymidazole, 4-aminomethylpyridine, 3-aminomethylpyridine, etc, preferably such that uncharged forms are obtained between pH 7 and 8.5 yet being significantly ionised at pH below 5. Cellulose is an example of a suitable solid support to which the ligand can be linked, but other solid supports should also work, e.g. regenerated cellulose, agarose, silica, coated silica, dextran, polymers (such as polyacrylates, polystyrene, polyacrylamide, polymethacrylamide), copolymers (such as copolymers of styrene and divinylbenzene), mixtures thereof and the like.

In one preferred embodiment, the HCIC resin for use according to the invention is MEP HyperCel, which is commercially available from Pall Corporation.

In certain embodiments, an HCIC resin as used herein is in the form of beads, that can for instance be used batch-wise or packed in columns. It is however to be understood that the term HCIC resin as used herein in other embodiments could also encompasses other physical forms such as sheets, for instance filters, which in that case comprise the HCIC functionality, analogous to for instance anion exchange filters which are filters that comprise an anion exchange functionality. It is for instance possible to couple the HCIC ligand (e.g. 4-MEP) to for instance Sartobind filters containing epoxy-groups (Sartorius, cat no 93EPOX068-DB-V), according to standard procedures, to obtain a filter comprising a HCIC functionality, which can be used as an HCIC resin according to the invention.

An advantage of HCIC is that high ionic strength is not required for hydrophobic binding of Factor XI to the resin. In certain preferred embodiments therefore, the biological fluid comprising Factor XI is contacted with the HCIC resin when this biological fluid has an ionic strength that is not higher than the ionic strength of a 0.5 M NaCl solution, preferably not higher than the ionic strength of a 250 mM NaCl solution. Procedures for determining the specific conductance of a solution are well known to those skilled in the art. A suitable biological fluid may for instance comprise 0-200 mM NaCl, e.g. 50 mM NaCl or 100 mM NaCl.

In preferred embodiments, the step of contacting the biological fluid containing coagulation Factor XI with the HCIC resin is carried out between pH 6 and 12, preferably between 6 and 9, more preferably between 6.5 and 8.5.

After binding the Factor XI to the HCIC resin, unbound and/or loosely bound contaminants are removed by washing the HCIC resin. Preferably, said washing step is performed using a solution having a pH between 5.5 and 12, more preferably between 6 and 9. Examples of suitable solutions for this washing step are water, Tris buffered saline (TBS), Phosphate buffered saline (PBS), and the like. In certain embodiments, a solution used during the washing step comprises benzoate or caprylate, e.g. 50 mM Na-benzoate or 25 mM Na-caprylate, for instance to remove albumin if present. In certain embodiments, a solution used during the washing step comprises a detergent, e.g. Tween-20, Tween-80, Triton X100, etc. In certain embodiments, a solution used during the washing step comprises caprylate or benzoate and a detergent, e.g. benzoate and Tween.

For elution of Factor XI, the HCIC resin containing the bound Factor XI is treated with a solution having a pH below 5.5, preferably a pH between 3 and 5.5, more preferably a pH of 4 to 5, still more preferably a pH of about from 4.5 to 5. In certain embodiments, a step gradient is used, while in other embodiments block elution is used. Any buffer or combination of buffers that will result in the desired pH drop can be used. Citrate or acetate buffers are examples of well suited elution solutions. Factor XI elutes between about pH 5.2 and 4.8 from MEP HyperCel. The elution buffer may comprise salt, e.g. 50 mM NaCl. In certain embodiments, the elution buffer also comprises a detergent, e.g. 0.02% Tween 80.

One advantage of the method of the invention is that albumin, if present, does not bind. Furthermore, antibodies, if present, will not co-elute with Factor XI. Further, the resin can be cleaned and sanitized with 0.5 M NaOH. Importantly, Factor XI is stable in elution buffer, so that pH adjustments are not necessary and the elution buffer is also the storage buffer. It is also an advantage that Factor XI is not activated into Factor XIa due to the HCIC step. The method is also suitable for the separation of different isoforms of Factor XI.

Several properties of Factor XI, as well as the possibility to use it for hcmostatic treatment, are for istance described in WO 2005/049070. The term "Factor XI" as used herein encompasses within its meaning human Factor XI, such as wild type human plasma Factor XI (SEQ ID NO: 1 in WO 2005/049070), or platelet-derived Factor XI (SEQ ID NO: 2 in WO 2005/049070), as well as natural allelic variations thereof, but also Factor XI variants containing one or more amino acid alterations by deletion, substitution or addition, and chemically modified Factor XI. Several examples of such molecules are given in WO 2005/049070. for instance, one or more amino acids of Factor XI, preferably not more than 30, more preferably not more than 20, still more preferably not more than 10, most preferably zero, one, two or three amino acids, may be replaced with other amino acids, or eliminated, or added. Typically, molecules that are at least 70%, preferably at least 80%, more preferably at least 90%, still more preferably at least 95% identical in amino acid sequence to either of SEQ ID NO: 1 or 2 of WO 2005/049070 is encompassed within the term Factor XI as used herein. In preferred embodiments, Factor XI has the amino acid sequence of wild type human plasma Factor XI. Factor XI according to the invention has the capability to decrease clotting time in a Factor XI-deficient plasma, as can be measured using routine methods known to the skilled person.

Factor XI is purified or captured according to the invention from a biological fluid. A biological fluid may be any fluid derived from or containing cells, cell components or cell products. Biological fluids include, but are not limited to cell cultures, cell culture supernatants, cell lysates, cleared cell lysates, cell extracts, tissue extracts, blood, plasma, serum, milk, urine, plant extracts and fractions thereof, all of which may also be homogenizates and filtrates, and fractions thereof, for instance collected by chromatography of unfractionated biological fluids.

Factor XI may be purified from a wide variety of biological fluids, including cell culture supernatants, which naturally produce Factor XI, but preferably of cells which have been genetically modified to produce Factor XI, such as mammalian cells (for instance CHO, BHK, HEK293, PER.C6 cells, and the like) transformed with DNA coding for Factor XI. Recombinant expression of Factor XI has been described (see e.g. Kemball-Cook et al, *supra;* Meijers et al, *supra;* WO 2005/049070) and can be performed using routine methods known to the skilled person. In one embodiment, the biological material is derived from human blood plasma. In a preferred embodiment, the biological material is material derived from a culture of cells in which Factor XI is recombinantly expressed, for instance a cell culture supernatant or cell-conditioned culture medium thereof. The biological fluid may be treated by use of a number of methods prior to application on the HCIC resin. Non-exhaustive examples of such methods are centrifugation and filtration.

Cell culture supernatants or cell conditioned culture medium in the capture step of Factor XI according to the invention may be derived from cells grown in the presence of serum such as fetal calf serum, or grown in serum free medium. Cell conditioned culture medium denotes a nutrient medium in which cells have been cultured and which contains cell products. When working with biological fluids containing cells, cell debris and the like it is preferred to first filter and/or (ultra)centrifuge the fluid to remove particulate contaminants. Recombinant Factor XI is secreted by the cells into the cell culture medium (cell-conditioned culture medium), or present in cell lysates, and can be separated according to the invention from other cell components, such as cell waste products, cell debris and proteins or other collected material.

Purification of Factor XI is the process of increasing the concentration of Factor XI (enriching) in a sample in relation to other components of said sample, resulting in an increase of the purity of Factor XI. The increase in purity of Factor XI may be followed by use of methods known in the art, such as for instance by use of SDS-PAGE, HPLC or clot activity assay. Purification is done to remove undesired contaminants, and therewith increasing the purity of the Factor XI. The term purified as used herein in relation to a protein does not refer only to absolute purity (such as a homogeneous preparation); instead, it refers to a protein that is relatively purer than in the natural environment. A step of purifying a protein thus relates to obtaining a protein preparation in which the protein is purer than in the biological material prior to the purification step, i.e. the preparation contains less contaminants, contaminants in this context including other proteins than Factor XI. In general, it is desired to obtain a high level of purity for a Factor XI preparation, but it will be clear that the HCIC step of the invention already will be advantageous when some degree of purification of Factor XI as compared to its purity in the original biological fluid is achieved. Further purification can suitably be performed by other techniques if desired. In preferred embodiments, the HCIC purification step of the invention gives a yield of at least 50%, more preferred at least 60%, still more preferred at least 70%, still more preferred at least 80% of the loaded Factor XI amount, while enriching the purity of Factor XI with at least a factor 1.5, preferably at least a factor 2, more preferably at least a factor 5, still more preferably at least a factor 10. The eluted Factor XI in preferred embodiments has a purity of higher than 50%, preferably higher than 60%, more preferably higher than 70%, still more preferably higher than 80%, for instance about 90% or higher.

The use of HCIC resin in the capture or purification of Factor XI can be scaled up, and can be suitably combined with other purification and/or concentration techniques such as (anion and/or cation) ion-exchange chromatography, (ligand, antibody, or other) affinity chromatography, hydrophobic interaction chromatography, hydroxyapatite chromatography, gel filtration, centrifugation, ultrafiltration, differential precipitation, and the like, to obtain fractions of Factor XI protein of increased purity. One or more of these other techniques may suitably be performed before and/or after the HCIC step of the invention. Such other techniques are well known and within the reach of the skilled person, see e.g. (Bioprocess Technology volume 9. 1990. Separation Processes in Biotechnology. Marcel Dekker, Inc., New York and Basel. Editor: Juan A Asenjo).

As is known to the skilled person, protein purification takes usually place in three phases: a capture step, in which the desired protein is isolated from a crude cell lysate or from cell culture medium, or from another biological fluid comprising the desired protein and contaminants; an intermediate step, in which proteins are isolated from contaminants similar in size and/or other physical/chemical properties; and finally a polishing step. Each purification stage has certain chromatography techniques and bead sizes that are best suited to a specific protein. The intermediate step generally requires high resolution for good separation of components. Generally, bead size correlates inversely with resolution, and smaller bead sizes are thus more appropriate at this stage. Adsorptive techniques, such as ion-exchange, affinity chromatography and hydrophobic interaction are generally used in the first two stages of purification. The HCIC step for purifying Factor XI is very suitable for the first or second step of purification.

Gel filtration (also called size exclusion) is generally reserved for the polishing step, in which a small, highly concentrated sample is applied to the column. Gel filtration separates globular proteins according to their molecular weights (see e.g. Amersham Biosciences, "Gel filtration. Principles and methods", 1998). In short, an aqueous solution containing at least one protein is, with help of an eluent (usually an aqueous solution), passed through a solid phase or matrix composed of bead containing a range of pore sizes, chosen on the basis of the size of the target protein. The liquid volume "seen" by the column consists of a mobile phase (the liquid surrounding the beads, or "void volume") and a stationary phase (the liquid contained within the beads' pores). Molecules that are too large to enter the bead pores will only come in contact with the void volume and will therefore elute first from the column. The smallest molecules will diffuse into the pores and come into contact with the total column volume and will elute last, with molecules of intermediate size eluting between the void volume and the total column volume. Gel filtration can be performed under a variety of physical and chemical conditions, and conditions for best separation are easily determined by routine methods known to the skilled person.

It is also described herein a process comprising a step of HCIC chromatography for purification of Factor XI, said process further comprising a step of gel filtration chromatography, preferably as a polishing step. The material comprising Factor XI eluted from the HCIC resin can be loaded without further modification onto the gel filtration column, but alternatively may also be subject to other chromatography steps and/or buffer-exchanged before subjecting it to gel filtration chromatography.

Ion exchange chromatography (see e.g. Amersham Biosciences (2004), "Ion Exchange Chromatography & Chromatofocusing, Principles & Methods" Cat,no, 11-0004-21) offers a great degree of control and specificity. Ion exchange chromatography relies on charge interactions between the protein of interest and the ion exchange matrix, which is generally composed of a solid support, such as agarose, dextran, cross-linked cellulose and agarose, and the like, covalently bound to a charged group. Charged groups are classified according to type (cationic and anionic) and strength (strong or weak); the charge characteristics of strong ion exchange media do not change with pH, whereas with weak ion exchange media, sample loading and capacity can change owing to loss of charge at varying pH, preventing protein binding. Examples of commonly used charged groups include diethylaminoethyl (DEAE; weakly anionic exchanger), carboxymethyl (weakly cationic exchanger), quaternary ammonium (strongly anionic exchanger), and methyl sulfonate (strongly cationic exchanger). Other charged groups are available as well. Ion exchange resins selectively bind proteins of opposite charge; that is, a negatively charged resin will bind proteins with a positive charge and vice versa. The technique in general takes place in five steps: equilibration of the column to pH and ionic conditions ideal for target protein binding; reversible adsorption of the sample to the column through counterion displacement; introduction of elution conditions that change the buffer's pH or ionic strength in order to displace bound proteins; elution of substances from the column in order of binding strength (weakly bound proteins are eluted first); and re-equllibration of the column for subsequent purifications. The skilled person can design ion exchange chromatography protocols such that the target protein is selectively bound to the column (allowing contaminants to pass through) or so that that contaminants adsorb and the target protein is excluded. In addition to resins that can be used in batch or to prepare columns, ion exchange can currently also be performed using high throughput ion exchange membranes/filters (i.e. a charged membrane or filter that contains ion exchange groups). Such ion exchange filters are known in the art and commercially available, e.g. from Pall (e.g. Mustang^{™} series) and from Sartorius (e.g. Sartobind series). Such filters can have advantages compared to ion exchange columns, for instance some or all of the following: (i) faster flow rates, (ii) higher binding capacity, (iii) higher protein recovery, (iv) no packing or cleaning validation required for clinical use, and (v) no lifetime issues or storage issues when disposable filter cartridges are used.

In certain embodiments, Factor XI is purified according to a method of the present invention, i.e. comprising a step of HCIC chromatography, the method further comprising an anion exchange step.

In one embodiment, an anion exchange step is used prior to the HCIC step of the invention. One suitable anion exchange material for such a step is DEAE sepharose. The biological fluid containing Factor XI is applied to the anion exchange resin, to which impurities (e.g. albumin) bind while Factor XI does not bind, so that the flow through contains a biological fluid with Factor XI and less impurities compared to the material that is applied to the anion exchange resin. Such a purified biological fluid containing Factor XI is preferably applied to the HCIC resin according to the invention, without further adaptation for further purification. Alternatively, other steps may be included before application to the HCIC resin, e.g. filtration, dialysis, re-buffering, other chromatography steps, and the like. Factor XI will bind to the HCIC resin and can be eluted according to the invention to obtain a purified Factor XI preparation.

Thus, in certain exemplary embodiments, the invention provides a method for purifying Factor XI from a biological fluid, the method comprising a step of bringing a biological fluid comprising Factor XI into contact with an anion exchange resin and collecting the non-bound material including the Factor XI to obtain a preparation enriched in Factor XI, the method further comprising a step of bringing said preparation comprising Factor XI into contact with an HCIC resin to bind Factor XI to the HCIC resin, washing the resin to remove contaminants, and eluting Factor XI by treating the HCIC resin with a solution having a pH that is 5.5 or lower to obtain a preparation further enriched in Factor XI, and preferably subjecting said further enriched preparation to gel filtration chromatography to obtain a purified Factor XI preparation. The purity of Factor XI in said purified Factor XI preparation is preferably at least 90%, more preferably at least 95%.

Alternatively, the HCIC step of the invention could also be performed prior to an anion exchange step, preferably using a strong anion exchanger, for instance to remove residual DNA. The HCIC step can for instance also be applied directly on for instance cell culture supernatants. The HCIC step will result in preparations that are enriched in Factor XI. Further purification steps may be performed thereafter.

Factor XI can be purified using a cation exchange step. For instance WO 2005/049070 and WO 2006/128497, describe suitable materials, buffers and conditions for purification of Factor XI using cation exchange chromatography. Cation exchange for purification of Factor XI can be combined with the methods according to the present invention. For instance, a cation exchange step may suitably be performed after an HCIC step of the invention, preferably using a strong cation exchanger such as for instance S-sepharose, to which the eluate of the HCIC step, enriched in Factor XI, can be applied without further adaptation. The cation exchanger will bind Factor XI, impurities can be washed away, and Factor XI can be eluted for instance with increased salt concentrations, to obtain preparations that are further enriched in Factor XI. The concentration of Factor XI can also be increased using cation exchange chromatography. Factor XI preparations resulting from a cation exchange step according to the invention can be subjected to gel filtration.

Thus, in certain exemplary embodiments, the invention provides a method for purifying Factor XI from a biological fluid, the method comprising a step of bringing a biological fluid comprising Factor XI into contact with an HCIC resin to bind Factor XI to the HCIC resin, washing the resin to remove contaminants, and eluting Factor XI by treating the HCIC resin with a solution having a pH that is 5.5 or lower to obtain a preparation enriched in Factor XI, the method further comprising a step of bringing said preparation comprising Factor XI into contact with a cation exchange resin to bind Factor XI, eluting Factor XI from said cation exchange resin to obtain a preparation further enriched in Factor XI, and preferably subjecting said further enriched preparation to gel filtration chromatography to obtain a purified Factor XI preparation.

It will be clear that different embodiments of the invention can be combined, for instance a method is provided wherein a biological fluid comprising Factor XI is brought into contact with an anion exchange resin, the non-bound material including Factor XI is subject to a HCIC step of the invention, resulting in a further enriched Factor XI preparation, which is subjected to cation exchange chromatography to result in an optionally further purified and/or concentrated Factor XI preparation, which is optionally subjected to gel filtration.

Different conditions such as buffers used during an ion exchange step to obtain enrichment of Factor XI using such a step may be employed and can routinely vary for instance column materials, salt concentrations and/or pH of the buffers to obtain optimal enrichment for Factor XI.

The practice of this invention will employ, unless otherwise indicated, conventional techniques of immunology, molecular biology, microbiology, cell biology, recombinant DNA, and protein purification, which are within the skill of the art. See e.g. Sambrook, Fritsch and Maniatis, Molecular Cloning: A Laboratory Manual, 2nd edition, 1989; Current Protocols in Molecular Biology, Ausubel FM, et al, eds, 1987; the series Methods in Enzymology (Academic Press, Inc.); PCR2: A Practical Approach, MacPherson MJ, Hams BD, Taylor GR, eds, 1995; Antibodies: A Laboratory Manual, Harlow and Lane, eds, 1988; Bioprocess Technology vol 9. Separation Processes in Biotechnology (Marcel Dekker, Inc), Juan A Asenjo, ed, 1990.

The invention is further explained in the following examples. The examples do not limit the invention in any way. They merely serve to clarify the invention.

### EXAMPLES

### Example 1: Purification of Factor XI by antibody affinity chromatography

Activity of several Factor XI preparations in the experiments was tested using a FXI clot assay. The principle of the FXI clot assay is based on prolonged activated partial tromboplastin time (aPTT) resulting from deficiency in FXI. The clotting activity of a FXI containing preparation is determined in FXI-deficient plasma by measuring the clotting time. In brief, FXI-deficient plasma is mixed with a dilution of plasma or another FXI-containing preparation and incubated with aPTT reagent (Pathromtin SL). The reaction is started by the addition of CaCl₂. The clotting time is measured on the Behring Coagulation Sytem and plotted against FXI concentration. If the preparation contains active Factor XI, the clotting time is lowered compared to negative control.

Wild type Factor XI was recombinantly produced in adherently cultured (culture medium: DMEM with 2-10% heat-inactivated calf serum) PER.C6® cells in roller bottles. The material was harvested and supplemented with Benzamidine (2 mM) pending purification. Factor XI was purified by means of an antibody column (using monoclonal antibody to human Factor XI, Lot: Mab HFXI 320, Kordia B.V., Leiden), according to the method described by (Sun Y, Gailani D. Identification of a factor IX binding site on the third apple domain of activated factor XI. J.Biol.Chem 1996; 271; 29023-29028). After binding and washing, Factor XI was eluted from the column with 2 M NaSCN. The protein containing fractions were pooled and buffer exchanged to TBS pH 7.5 (50 mM Tris, 150 mM NaCl, 2 mM Benzamidine). FXI could be purified with this method at a yield of 20-40%. However, the purity was not higher than about 50-60%, and the clotting activity after re-buffering (to remove chaotropic agent) appeared lower than that of plasma.

Elutions with 3 M NaSCN and 4 M Guanidine resulted in higher yields, but decreased purities of FXI.

### Example 2: Purification of Factor XI by HCIC

50 ml supernatant (of a culture of PER.C6 cells that express wt FXI) containing Factor XI was batchwise incubated (pH 7-8) with 1 ml MEP HyperCel resin (Pall Biosepra, catalogue number 12035-010) on a roller bank for 2 hours. The resin was then packed in a column (volume 1 ml, 0.5 cm ID, 5 cm length) and flushed with TBS (50 mM Tris pH 7.5, 150 mM NaCl, 2 mM Benzamidine). Factor XI was eluted from the column with 50 mM Na-citrate pH 4. All Factor XI that was bound to the MEP HyperCel, could be recovered. The eluate fraction was run on a 4-12% Bis-Tris SDS-PAGE gel both under reducing and non-reducing conditions, followed by Western Blotting. Single bands were obtained under both circumstances, indicating that Factor XI is not destroyed or activated by the purification method. Factor XI thus purified showed activity in a clotting assay.

Thus, Factor XI can bind to MEP HyperCel at pH 7.5 and can be quantitatively eluted in intact form at pH 4.

### Example 3: different conditions for HCIC purification of Factor XI

Example 2 shows that FXI can bind to a HCIC resin and can be eleted therefrom. In this example, several different conditions were tested: the salt concentrations in the buffers were varied, citrate and acetate were tested in the elution buffers, and different (pH-) step gradients were used. A packed column with I ml MEP HyperCel was used in this experiment.
The buffers used were:
- Equilibration Buffer, pH 7.4: 50 mM Tris; 0, 50 or 100 mM NaCl; 0 or 2 mM benzamidine
- Wash Buffer 1: distilled water
- Wash Buffer 2, pH 7.4: 50 mM Tris; 0, 50 or 100 mM NaCl, 25 mM Na-caprylate
- Elution Buffer 1, pH 6: 50 mM Na-citrate or Na-acetate; 0, 50 or 100 mM NaCl
- Elution Buffer 2, pH 3: 50 mM Na-citrate or Na-acetate; 0, 50 or 100 mM NaCl
- Cleaning Buffer: 0.5 M NaOH
The procedure used is as follows:
The flowrate applied was 0.5 ml/min (150 cm/hr). First, the column was equilibrated with 5 column volumes equilibration buffer, then 50 ml harvest containing Factor XI was loaded on the column, and the flowthrough fraction was collected to detect any breakthrough of Factor XI.
   Wash step 1: after the product was loaded, the column was washed with 10 column volumes (CVs) Wash Buffer 1.
   Wash step 2: after Wash 1 the column was washed with 10 CVs Wash Buffer 2. Elution step: Factor XI was eluted from the column with citrate or acetate buffer by applying a (pH-)step gradient.
Two kind of step gradients were used as follows:
(i) A shallow step gradient (7-step):
   10 CVs 100% Elution Buffer 1 (pH = 6)
   5 CVs 88% Elution Buffer 1, 12% Elution Buffer 2 (pH = 5.64)
   5 CVs 76% Elution Buffer 1,24% Elution Buffer 2 (pH = 5.28)
   5 CVs 64% Elution Buffer 1,36% Elution Buffer 2 (pH = 4.92)
   5 CVs 52% Elution Buffer 1, 48% Elution Buffer 2 (pH = 4.56)
   5 CVs 36% Elution Buffer 1,64% Elution Buffer 2 (pH = 4.08)
   5 CVs 100% Elution Buffer 2 (pH = 3)
(ii) Alternatively, a steep gradient (3-step) was used:
   10 CVs 98% Elution Buffer 1,2% Elution Buffer 2 (pH = 5.94)
   10 CVs 52% Elution Buffer 1,48% Elution Buffer 2 (pH = 4.56)
   10 CVs 100% Elution Buffer 2 (pH = 3)
After the elution step, the column is cleaned with 10 CVs Cleaning Buffer, and reequilibrated with 5 CVs Equilibration Buffer for the next run. The results of the tested conditions are shown in Table 1.

**Table 1. Yield of FXI purified on MEP HyperCel using different conditions (50 ml crude harvest loadcd on 1 ml column)**

| **Description of process** | **Applied (µg)** | **Bound to MEP (µg)** | **Obtained (µg)** | **Yield (%)** |
|---|---|---|---|---|
| 7-Step Gradient, Citrate elution | 388 | 385 | 152 | 39 |
| 7-Step Gradient, Citrate elution, 50 mM NaCl | 315 | 312 | 229 | 73 |
| 3-Step Gradient, Citrate elution, 50 mM NaCl | 360 | 360 | 291 | 81 |
| 3-Step Gradient, Citrate elution, 100 mM NaCl | 327 | 327 | 193 | 59 |
| 3-Step Gradient, Citrate elution, 100 mM NaCl, washsteps reversed | 327 | 327 | 193 | 52 |
| 7-Step Gradient, Acetate elution, 50 mM NaCl | 422 | 417 | 291 | 70 |
| 7-Step Gradient, Acetate elution, 100 mM NaCl | 570 | 570 | 402 | 81 |
| 3-Step Gradient, Acetate elution, 50 mM NaCl | 475 | 470 | 442 | 94 |

Factor XI could thus be purified with both citrate and acetate buffers, and both with 50 mM and 100 mM NaCl concentrations. In further experiments, 3-step gradients were used and buffers contained 50 mM NaCl. In some cases, a 4-step gradient, which is the 3-step gradient with as additional (third) step 40% Elution Buffer 1 and 60% of Elution Buffer 2 (pH = 4.2), was used.

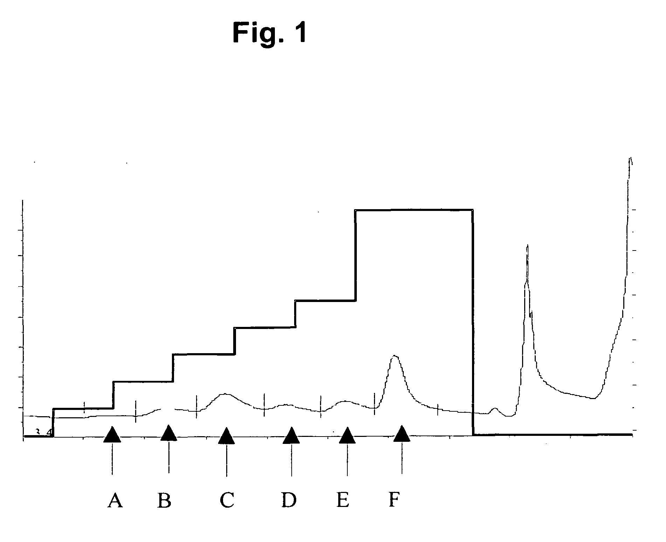

Fig. 1 shows a gradient profile of the purification of a Factor XI preparation on a 1 ml MEP Hypercel column, using a 7-step gradient (citrate, 50 mM NaCl). Factor XI peaks are indicated with arrows.

It was observed that by applying a shallow gradient, multiple FXI peaks were obtained. This suggests that it is possible to seperate different isoforms of FXI with MEP HyperCel. Such isoforms differ in charge, probably due to difference in sialic acid content.

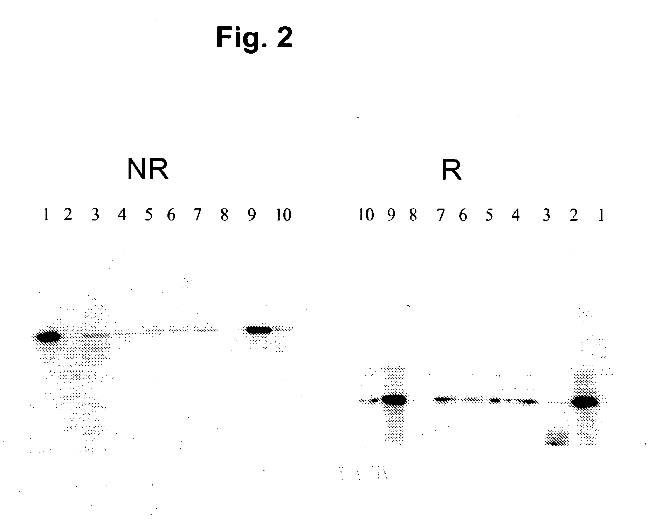

The eluate fraction was run under reducing and non-reducing conditions on a 4-12% Bis-Tris gel, followed by Western Blotting (Fig. 2). Single bands were obtained under both circumstances, indicating that Factor XI is not destroyed or activated by the purification method.

The Factor XI fractions eluted from the MEP HyperCel column, when tested were more than 85% pure estimated on SDS-PAGE, and showed activity in a clotting assay.

This example shows that Factor XI can be purified from a cell culture medium from cells that recombinantly express Factor XI in the presence of serum, using HCIC and a variety of different buffer conditions.

### Example 4: HCIC purification of Factor XI from serum free culture

In this example Factor XI was purified from cell culture harvest grown under serum free conditions. Factor XI was eluted from MEP HyperCel by means of a 3-step or 4-step pH gradient (pH 5.5-3) with 50 mM citrate, 50 mM NaCl buffers as mentioned in Example 3.

Factor XI could be purified from this material in this way. The yield obtained is however lower then with harvest containing serum. The yield could be restored by adding Tween to all the buffers. This yield increase was seen with all the concentrations tested between 0.02 and 0.1 % of Tween 80.

A representative example of a run on MEP HyperCel of this example is shown in Fig. 3. Factor XI elutes in the fraction of 52% Elution Buffer 1, 48% Elution Buffer 2 (pH around 4.5).

The purity, estimated on SDS-PAGE, of the samples thus obtained was higher than 95%.

This example shows that Factor XI can be purified from serum-free cultures of cells that recombinantly express Factor XI.

### Example 5: HCIC purification of Factor XI from human plasma

In this example DEAE-treated Human Plasma was used as the source for Factor XI. The treatment with DEAE results in removal of several contaminants, including albumin, while Factor XI does not bind to this material and is thus still present in the plasma fraction. This material was applied to a MEP HyperCel column.

Factor XI was eluted from MEP Hypercel by means of a 3-step pH gradient (pH 6-3, citrate buffer, 50 mM NaCl) as described in example 3.

It was possible to enrich for Factor XI in this way, but IgG's co-eluted from the column, resulting in a relatively low purity for Factor XI.

Therefore, 25 ml DEAE treated human plasma was subjected to a 1 ml Protein G column, to which IgG's selectively bound while more than 99% of the loaded Factor XI appeared in the flowthrough. Thus, a biological fluid containing Factor XI and depleted of IgG's was obtained.

This material was loaded onto a 1 ml MEP HyperCel column. A 4-step gradient was used as described in Example 3. Factor XI appeared in the eluate fraction with 52% Elution Buffer 1 and 48% Elution Buffer 2 (pH about 4.5). Fig. 4 shows the gradient profile. From this step, Factor XI was obtained with a yield of 75%, and had a purity of about 75% as estimated on SDS-PAGE.

This example shows that an anion exchange column can be used prior to the HCIC step of the invention to purify Factor XI, and shows that IgG's can be depleted from a sample prior to the HCIC column by a Protein G column so that the IgG's do not interfere with (give impurities in) the Factor XI elution from the HCIC column. Further, this example demonstrates that Factor XI can be purified from plasma using HCIC.

### Example 6: Gel filtration of Factor XI fraction obtained using HCIC

In this example a 10 ml Factor XI fraction, purified with MEP HyperCel as described above (from a serum-containing cell culture supernatant), was buffer exchanged to the final storage buffer by means of size exclusion over a 30 ml Sephadex 75 column. (16 mm ID x 15 cm height). The column was equilibrated with the storage buffer of choice, in our case 50 mM Na-citrate, 150 mM NaCl, pH5.5, 0.02% Tween 80.

In this way Factor XI was further polished and buffer exchanged in one step.

The elution profile is shown in Fig. 5. The purity of Factor XI thus obtained was higher than 90% as estimated by SDS-PAGE.

## Claims

1. A method for purifying Factor XI from a biological fluid, comprising the steps of:
a) contacting the biological fluid containing Factor XI with an Hydrophobic Charge Induction Chromatography (HCIC) resin to bind Factor XI to said HCIC resin; wherein said HCIC resin is hydrophobic at pH 7-8.5 and is positively charged at pH below 5;
b) washing the HCIC resin with a solution having a pH between 5.5 and 12 to remove contaminants; and
c) eluting Factor XI by treating the HCIC resin with a solution having a pH that is 5.5 or lower.

2. A method according to claim 1, wherein the HCIC resin is a 4-mercapto-ethyl-pyridine linked to a cellulose matrix.

3. A method according to any one of the preceding claims, wherein step a) is carried out at a pH between 6 and 9.

4. A method according to any one of the preceding claims, wherein step b) comprises washing with a solution having a pH between 6 and 9.

5. A method according to claim 4, wherein said solution having a pH of between 6 and 9 comprises caprylate.

6. A method according to any one of the preceding claims, wherein step c) is carried out using a solution that has a pH between 5.5 and 3.

7. A method according to any one of the preceding claims, further comprising at least one anion exchange step.

8. A method according to any one of the preceding claims, wherein the biological fluid is chosen from blood plasma, cell culture supernatants, cell-conditioned culture medium and fractions derived from an earlier chromatographic separation step.

9. A method according to any one of the preceding claims, wherein said Factor XI has been recombinantly expressed.

10. Use of a Hydrophobic Charge Induction Chromatography (HCIC) resin for the purification of Factor XI from a biological fluid.

## Patentansprüche

1. Verfahren zur Aufreinigung von Faktor XI aus einer biologischen Flüssigkeit, umfassend die Schritte:
a) Inkontaktbringen der biologischen Flüssigkeit, die Faktor XI enthält, mit einem hydrophobe Ladungsinduktions-Chromatographie (HCIC)-Harz, um Faktor XI an das HCIC-Harz zu binden; wobei das HCIC-Harz bei pH 7-8.5 hydrophob ist und bei einem pH-Wert unter 5 positiv geladen ist;
b) Waschen des HCIC-Harzes mit einer Lösung, die einen pH-Wert zwischen 5.5 und 12 hat, um Verunreinigungen zu beseitigen; und
c) Eluieren von Faktor XI durch Behandlung des HCIC-Harzes mit einer Lösung, die einen pH-Wert von 5.5 oder niedriger hat.

2. Verfahren nach Anspruch 1, wobei das HCIC-Harz ein an eine Cellulosematrix gebundenes 4-Mercapto-ethyl-pyridin ist.

3. Verfahren nach einem der vorangegangenen Ansprüche, wobei Schritt a) bei einem pH-Wert zwischen 6 und 9 ausgeführt wird.

4. Verfahren nach einem der vorangegangenen Ansprüche, wobei Schritt b) das Waschen mit einer Lösung, die einen pH-Wert zwischen 6 und 9 hat, umfasst.

5. Verfahren nach Anspruch 4, wobei die Lösung, die einen pH-Wert zwischen 6 und 9 hat, Caprylat umfasst.

6. Verfahren nach einem der vorangegangenen Ansprüche, wobei Schritt c) unter Verwendung einer Lösung, die einen pH-Wert zwischen 5.5 und 3 hat, ausgeführt wird.

7. Verfahren nach einem der vorangegangenen Ansprüche, weiter umfassend mindestens einen Anionenaustausch-Schritt.

8. Verfahren nach einem der vorangegangenen Ansprüche wobei die biologische Flüssigkeit ausgewählt ist aus Blutplasma, Zellkulturüberständen, zellkonditioniertem Kulturmedium und Fraktionen, die von einem früheren chromatographischen Auftrennungsschritt stammen.

9. Verfahren nach einem der vorangegangenen Ansprüche, wobei der Faktor XI rekombinant exprimiert wurde.

10. Verwendung eines hydrophobe Ladungsinduktions-Chromatographie (HCIC)-Harzes zur Aufreinigung von Faktor XI aus einer biologischen Flüssigkeit.

## Revendications

1. Procédé de purification du facteur XI d'un liquide biologique, comprenant les étapes de :
a) mise en contact du liquide biologique contenant le facteur XI avec une résine de chromatographie d'induction de charges hydrophobes (HCIC) pour lier le facteur XI à ladite résine HCIC ; ladite résine HCIC étant hydrophobe au pH 7 à 8,5 et étant positivement chargée au pH en dessous de 5;
b) le lavage de la résine HCIC avec une solution ayant un pH compris entre 5,5 et 12 pour retirer les substances contaminantes ; et
c) l'élution du facteur XI par traitement de la résine HCIC avec une solution ayant un pH qui est de 5,5 ou moins.

2. Procédé selon la revendication 1, la résine HCIC étant une 4-mercapto-éthyl-pyridine liée à une matrice de cellulose.

3. Procédé selon l'une quelconque des revendications précédentes, l'étape a) étant conduite à un pH compris entre 6 et 9.

4. Procédé selon l'une quelconque des revendications précédentes, l'étape b) comprenant le lavage avec une solution ayant un pH compris entre 6 et 9.

5. Procédé selon la revendication 4, dans lequel ladite solution ayant un pH compris entre 6 et 9 comprend du caprylate.

6. Procédé selon l'une quelconque des revendications précédentes, ladite étape c) étant conduite en utilisant une solution ayant un pH compris entre 5,5 et 3.

7. Procédé selon l'une quelconque des revendications précédentes, comprenant en outre au moins une étape d'échange d'anions.

8. Procédé selon l'une quelconque des revendications précédentes, le liquide biologique étant choisi parmi le plasma sanguin, les surnageants de culture cellulaire, les milieux de culture conditionnés par des cellules et des fractions dérivées d'une étape antérieure de séparation chromatographique.

9. Procédé selon l'une quelconque des revendications précédentes, ledit facteur XI ayant été exprimé de manière recombinante.

10. Utilisation d'une résine de chromatographie d'induction de charges hydrophobes (HCIC) pour la purification du facteur XI d'un liquide biologique.
